# EUROPÄISCHE PATENTSCHRIFT

(11) **EP 0 407 844 B1**
(45) Veröffentlichungstag und Bekanntmachung des Hinweises auf die Patenterteilung: **06.04.1994**
(21) Anmeldenummer: 90112539.3
(22) Anmeldetag: 30.06.1990
(51) Int. Cl.: C07D 209/14, A61K 31/40

(54) **Indolderivate**
Indole derivatives
Dérivés de l'indole

(30) Priorität: 13.07.1989 DE 3923045
(43) Veröffentlichungstag der Anmeldung: 16.01.1991
(73) Patentinhaber: MERCK PATENT GmbH, D-64271 Darmstadt (DE)
(72) Erfinder: Böttcher, Henning, Dr., D-6100 Darmstadt (DE); Seyfried, Christoph, Dr., D-6104 Seeheim-Jugenheim (DE); Greiner, Hartmut, Dr., D-6100 Darmstadt (DE)

(56) Entgegenhaltungen:
- BE-A- 771 285
- US-A- 3 929 793
- US-A- 4 089 853

## Beschreibung

Die Erfindung betrifft die neue Verbindung 3-[4-(4-p-Methoxyphenyl-piperazino)-butyl]-5-methoxy-indol (I) sowie deren Salze.

In der BE-A-771285 (Derwent 12863 T) findet sich eine allgemeine Formel 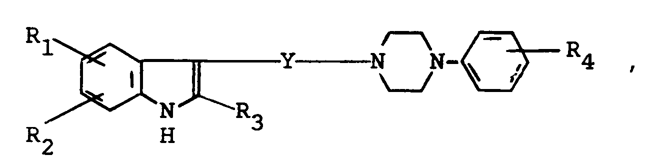
worin R₁ und R₄ jeweils auch Methoxy, R₂ und R₃ jeweils auch H und Y auch -(CH₂)₄- bedeuten können.

Ferner ist in der NL-A-67 13 659 (Derwent 31 565 F) eine allgemeine Formel angeben. 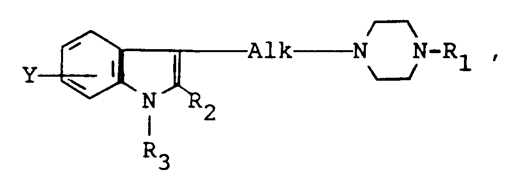
worin R₁ auch p-Methoxyphenyl, R² und R³ jeweils auch H, Y auch Methoxy und Alk auch -(CH₂)₄- bedeuten können.

Die spezielle Verbindung I ist jedoch in beiden Druckschriften nicht genannt, so daß sie demgegenüber neu ist.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Im einzelnen hemmen sie die Bindung von tritiierten Serotoninliganden an hippocampale Rezeptoren (Cossery et al., European J. Pharmacol. 140 (1987), 143-155). Außerdem treten Veränderungen der 5-HTP-Akkumulation im N.raphe auf (Seyfried et al., European J. Pharmacol. 160 (1989), 31-41) und in anderen Hirngebieten wie Cortex, Hippocampus, Striatum und Hypothalamus. Demgemäß können die Verbindungen anxiolytische (Söderpalm et al., Pharmacol. Biochem. Behavior 32 (1989), 259-265), antipsychotische (Ahlenius, Pharmacol. and Toxicol. 64 (1989), 3-5), antidepressive (Cervo und Samanin, Europ. J. Pharmacol. 144 (1987), 223-229) und anorektische (Aulakh et al., Europ. J. Pharmacol. 146 (1988), 253-259) Wirkungen entfalten. Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt.

Die Verbindung I und ihre physiologisch unbedenklichen Säureadditionssalze können daher als Arzneimittelwirkstoffe für Anxiolytika, Antidepressiva, Neuroleptika und/oder Antihypertonika und auch als Zwischenprodukte zur Herstellung anderer Arzneimittelwirkstoffe verwendet werden.

Gegenstand der Erfindung sind das Indolderivat I sowie seine Salze, ferner ein Verfahren zur Herstellung dieser Verbindungen, dadurch gekennzeichnet, daß man ein 3-(4-X¹-butyl)-5-methoxy-indol (II) worin
- X¹: X oder NH₂ und
- X: Cl, Br, J, OH oder eine reaktionsfähig funktionell abgewandelte OH-Gruppe bedeuten
mit einer Verbindung der Formel III

X²-CH₂CH₂CH(p-CH₃O-C₆H₄)-CH₂CH₂X³ III

worin
- X² und X³: gleich oder verschieden sein können und, falls X¹ = NH₂ ist, jeweils X, andernfalls zusammen NH bedeuten
umsetzt
oder daß man eine sonst I entsprechende Verbindung, die jedoch an Stelle eines oder mehrerer Wasserstoffatome eine oder mehrere reduzierbare oder hydrogenolytisch abspaltbare Gruppe(n) und/oder eine oder mehrere zusätzliche C-C- und/oder C-N-Bindung(en) enthält, mit einem reduzierenden Mittel behandelt
oder daß man eine sonst I entsprechende Verbindung, die jedoch an Stelle eines Wasserstoffatoms eine solvolytisch abspaltbare Gruppe(n) enthält, mit einem solvolysierenden Mittel behandelt
und/oder daß man eine erhaltene Base I durch Behandeln mit einer Säure in eines ihrer Salze umwandelt und/oder aus einem ihrer Salze durch Behandeln mit einer Base freisetzt.

Die Herstellung der Verbindung I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York; DE-OS 33 42 632) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu der Verbindung I umsetzt.

In den Indolderivaten der Formel II ist X¹ vorzugsweise X; dementsprechend sind in den Verbindungen der Formel III X² und X³ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend ist das Indolderivat I insbesondere durch Umsetzung von 3-(4-Chlorbutyl)- oder 3-(4-Brombutyl)-5-methoxy-indol mit 1-p-Methoxyphenylpiperazin (IIIa) erhältlich.

Die Verbindungen der Formeln II und insbesondere III sind zum Teil bekannt; die nicht bekannten Verbindungen der Formeln II und III können leicht analog zu den bekannten Verbindungen hergestellt werden. 3-(4-Hydroxybutyl)-5-methoxy-indol ist z.B. durch Reduktion von 4-(5-Methoxy-3-indolyl)-buttersäure oder ihrer Ester erhältlich. Behandeln mit Thionylchlorid, Bromwasserstoff, Phosphortribromid oder ähnlichen Halogenverbindungen liefert die entsprechenden Halogenide. Die entsprechenden Sulfonyloxyverbindungen sind zugänglich aus dem Alkohol durch Umsetzung mit den entsprechenden Sulfonsäurechloriden. 3-(4-Jodbutyl)-5-methoxy-indol ist z.B. durch Einwirkung von Kaliumjodid auf den zugehörigen p-Toluolsulfonsäureester erhältlich. 3-(4-Aminobutyl)-5-methoxy-indol ist z.B. aus den Halogeniden mit Phthalimidkalium oder durch Reduktion der entsprechenden Nitrile herstellbar.

Die Ausgangsverbindung IIIa ist bekannt und z.B. erhältlich durch Umsetzung von p-Methoxyanilin mit Bis-(2-chlorethyl)-amin. Verbindungen der Formel III (X² und X³ = jeweils X) sind z.B. herstellbar durch Reduktion von Diestern der Formel AlkylOOC-CH₂-NAr-CH₂-COOAlkyl zu Diolen der Formel HO-CH₂CH₂-NAr-CH₂-CH₂OH (III, X² = X³ = OH) und gegebenenfalls anschließende Umsetzung mit SOCl₂ bzw. PBr₃ (Ar = p-Methoxyphenyl).

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente (3-(4-Aminobutyl)-5-methoxy-indol bzw. IIIa) kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel IV

Ind-L-W IV

worin
- Ind: einen 5-Methoxy-3-indolyl-rest, der zusätzlich durch eine Arylsulfonylgruppe oder eine Benzylgruppe in 1-Stellung substituiert sein kann,
- L: -(CH₂)₄- oder eine dem Rest -(CH₂)₄- entsprechende Kette, worin jedoch eine oder mehrere -CH₂-Gruppe(n) durch -CO- und/oder ein oder mehrere Wasserstoffatome durch OH-Gruppen ersetzt sind,
- W: 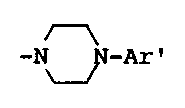 und
- Ar′: eine p-Methoxyphenylgruppe, die zusätzlich ein- oder zweifach durch Cl oder Br substituiert sein kann, bedeuten,
worin jedoch nicht gleichzeitig Ind = 5-Methoxy-3-indolyl, L = -(CH₂)₄- und W = p-Methoxyphenyl-piperazino sein können.

In den Verbindungen der Formel IV ist L bevorzugt -(CH₂)₃-CO-, aber auch -CO-(CH₂)₂-CO-, -CO-(CH₂)₃-, -CH₂-CO-CH₂CH₂- oder CH₂-CH₂-CO-CH₂-.

Verbindungen der Formel IV sind z.B. herstellbar durch Umsetzung von IIIa mit einer Verbindung der Formel V

Ind-L-X¹ V

worin
- Ind, L und X¹: die oben angegebenen Bedeutungen haben,
unter den Bedingungen, die oben für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol. Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen 0 und 100°.

Besonders vorteilhaft lassen sich -CO-Gruppen in Säureamiden [z.B. 4-(5-Methoxy-3-indolyl)-buttersäure-(N′-p-methoxyphenyl-piperazid)] mit LiAlH₄ in THF bei Temperaturen zwischen 0 und 66° zu CH₂-Gruppen reduzieren. Dabei können in 1-Stellung des Indolring befindliche Arylsulfonyl-Schutzgruppen gleichzeitig reduktiv abgespalten werden.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel 3-4 Stunden gekocht. Anschließend wird das Wasser abdestilliert und das gebildete Hydrazon bei Temperaturen bis zu 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine solvolytisch abspaltbare Gruppe enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit 1-Z-3-(4-X-butyl)-5-methoxy-indolen (worin Z eine solvolytisch abspaltbare Gruppe ist und X die angegebene Bedeutung hat). So können insbesondere 1-Acylindolderivate (entsprechend I, aber in 1-Stellung des Indolrests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu I hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Eine erhaltene Base I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freie Base I kann, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindung I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend die Verdung I und/oder eines ihrer physiologisch unbedenklichen Salze. Diese Zübereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindung I und ihre physiologisch unbedenklichen Salze können bei der therapeutischen Behandlung des menschlichen oder tierischen Körpers und bei der Bekämpfung von Krankheiten verwendet werden, insbesondere von extrapyramidalen Störungen bei der Neuroleptikatherapie, von Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, weiterhin zur Therapie cerebraler Störungen (z.B. Migräne), insbesondere in der Geriatrie ähnlich wie gewisse Ergot-Alkaloide.

Dabei werden die erfindungsgemäßen Substanzen in der Regel in Analogie zu bekannten Präparaten [z.B. GR 43175; vgl. Humphrey et al., Br. J. Pharmacol. 94 (1988), 1123-1132] verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (0,2 bis 1 mg pro Dosierungseinheit; 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

### Beispiel 1

Man rührt eine Lösung von 23,75 g 3-(4-Chlorbutyl)-5-methoxy-indol und 19,2 g IIIa in 250 ml Acetonitril 12 Std. bei 20°, arbeitet wie üblich auf und erhält I; Monohydrochlorid, F. 236-237,5°. Methansulfonat, F. 180-181°. Succinat, F. 154°. Fumarat, F. 181°. Maleat, F. 163°.

### Beispiel 2

Ein Gemisch von 2,18 g 3-(4-Aminobutyl)-5-methoxy-indol und 2,48 g N,N-Bis-(2-chlorethyl)-p-methoxyanilin in 40 ml Aceton und 40 ml Wasser wird 24 Std. gekocht und wie üblich aufgearbeitet. Man erhält I.

### Beispiel 3

Zu einer Suspension von 11,7 g LiALH₄ in 1000 ml absolutem THF tropft man unter Rühren und N₂-Atmosphäre eine Suspension von 40,7 g 4-(5-Methoxy-3-indolyl)-buttersäure-(N′-p-methoxyphenyl-piperazid) [F. 171-174; erhältlich aus 4-(5-Methoxy-3-indolyl)-buttersäure und IIIa in Gegenwart von Carbonyldiimidazol in THF bei 20°] in 3 l absolutem THF, kocht 1 Std., kühlt ab, zersetzt mit Wasser und Natronlauge, arbeitet wie üblich auf und erhält I.

### Beispiel 4

Zu einer Suspension von 13,5 g 4-(5-Methoxy-3-indolyl)-buttersäure-(N′-p-methoxyphenyl-piperazid) in 200 ml THF gibt man unter Rühren und Einleiten von N₂ 23,2 ml einer 70%igen Lösung von Natrium-bis-(2-methoxyethoxy)-aluminiumdihydrid in Toluol. Dabei steigt die Temperatur auf 40°. Man rührt noch 16 Std., arbeitet wie üblich auf und erhalt I.

### Beispiel 5

Man kocht 5,19 g 1-Benzolsulfonyl-3-[4-(4-p-methoxyphenyl-piperazino)-butyl]-5-methoxy-indol [erhältlich aus 1-Benzolsulfonyl-3-(4-chlorbutyl)-5-methoxy-indol und IIIa] mit 1 g KOH in 7 ml Wasser und 14 ml Ethanol 16 Std., arbeitet wie üblich auf und erhält I.

Die nachstehenden Beispiele betreffen pharmazeutische Zubereitungen, die die Verbindung I oder ihre Säureadditionssalze enthalten:

### Beispiel A: Tabletten

Ein Gemisch von I-Monohydrochlorid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

2 kg I-Methansulfonat werden in üblicher Weise in Hartgelatinekapseln gefüllt, so daß jede Kapsel 20 mg des Wirkstoffs enthält.

### Beispiel D: Ampullen

Eine Lösung von 1 kg I-Methansulfonat in 300 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. 3-[4-(4-p-Methoxyphenyl-piperazino)-butyl]-5-methoxyindol (I) und dessen Salze.

2. Verfahren zur Herstellung von 3-[4-(4-p-Methoxyphenylpiperazino)-butyl]-5-methoxyindol (I) sowie von dessen Salzen, dadurch gekennzeichnet, daß man ein 3-(4-X¹-butyl)-5-methoxy-indol (II) worin
X¹ X oder NH₂ und
X Cl, Br, J, OH oder eine reaktionsfähig funktionell abgewandelte OH-Gruppe bedeuten
mit einer Verbindung der Formel III
X²-CH₂CH₂CH(p-CH₃O-C₆H₄)-CH₂CH₂X³ III
worin
X² und X³ gleich oder verschieden sein können und, falls X¹ = NH₂ ist, jeweils X, andernfalls zusammen NH bedeuten
umsetzt oder daß man eine sonst I entsprechende Verbindung, die jedoch an Stelle eines oder mehrerer Wasserstoffatome eine oder mehrere reduzierbare oder hydrogenolytisch abspaltbare Gruppe(n) und/oder eine oder mehrere zusätzliche C-C- und/oder C-N-Bindung(en) enthält, mit einem reduzierenden Mittel behandelt
oder daß man eine sonst I entsprechende Verbindung, die jedoch an Stelle eines Wasserstoffatoms eine solvolytisch abspaltbare Gruppe(n) enthält, mit einem solvolysierenden Mittel behandelt
und/oder daß man eine erhaltene Base I durch Behandeln mit einer Säure in eines ihrer Salze umwandelt und/oder aus einem ihrer Salze durch Behandeln mit einer Base freisetzt.

3. Verfahren zur Herstellung pharmazeutischer Zubereitungen, dadurch gekennzeichnet, daß man die Verbindung I nach Patentanspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

4. Pharmazeutische Zubereitung, gekennzeichnet durch einen Gehalt an der Verbindung I nach Patentanspruch 1 und/oder einem ihrer physiologisch unbedenklichen Salze.

5. Verwendung der Verbindung I nach Patentanspruch 1 oder von deren physiologisch unbedenklichen Salzen zur Herstellung eines Arzneimittels.

## Claims

1. 3-[4-(4-p-Methoxyphenylpiperazino)butyl]-5-methoxyindole (I) and the salts thereof.

2. Process for the preparation of 3-[4-(4-p-methoxyphenylpiperazino)butyl]-5-methoxyindole (I) and of the salts thereof, characterized in that a 3-(4-X¹-butyl)-5-methoxyindole (II)
wherein
X¹ is X or NH₂, and
X is Cl, Br, I, OH or an OH group functionally modified to form a reactive group,
is reacted with a compound of formula III
X²-CH₂CH₂CH(p-CH₃O-C₆H₄)-CH₂CH₂X³ III
wherein
X² and X³ can be identical or different and are each X if X¹ = NH₂ or are together NH in other cases,
or in that a compound which conforms to I except that one or more hydrogen atoms have been replaced by one or more reducible or hydrogenolytically cleavable groups and/or one or more additional C-C and/or C-N bonds is treated with a reducing agent,
or in that a compound which conforms to I except that one hydrogen atom has been replaced by a solvolytically cleavable group is treated with a solvolysing agent, and/or in that a resultant base I is converted into one of its salts by treatment with an acid and/or liberated from one of its salts by treatment with a base.

3. A process for the manufacture of pharmaceutical preparations, characterized in that compound according to Claim 1 and/or one of its biocompatible salts are converted into a suitable dosage form together with at least one solid, liquid or semiliquid excipient or adjunct.

4. A pharmaceutical preparation, characterized in that it contains compound I according to Claim 1 and/or one of its biocompatible salts.

5. Use of compound I according to Claim 1, or its biocompatible salts, for the manufacture of a drug.

## Revendications

1. 3-[4-(4-p-méthoxyphénylpipérazino)butyl]-5-méthoxyindole (I) et ses sels.

2. Procédé de préparation du 3-[4-(4-p-méthoxyphénylpipérazino)butyl]-5-méthoxyindole (I), aussi bien que de ses sels, caractérisé en ce qu'on fait réagir un 3-(4-X¹-butyl)-5-méthoxyindole (II) dans lequel
X¹ représente X ou NH₂ et
X représente Cl, Br, I, OH ou un radical OH modifié fonctionnellement et réactif,
avec un composé de la formule III
X²-CH₂CH₂CH(p-CH₃O-C₆H₄)-CH₂CH₂X³ III
dans lequel
X² et X³ peuvent être identiques ou différents, et dans le cas où X¹ = NH₂, représentent chaque fois X, ou représentent sinon ensemble NH,
ou qu'on traite un composé correspondant à I, qui contient toutefois à la place d'un ou de plusieurs atome(s) d'hydrogène un ou plusieurs radical(ux), réductible(s) ou séparable(s) par hydrogénolyse et/ou une ou plusieurs liaison(s) à C-C et/ou C-N additionnelle(s), avec un moyen réducteur,
ou qu'on traite un composé correspondant à I, qui toutefois contient à la place d'un atome d'hydrogène un radical séparable par solvolyse, avec un moyen de solvolyse,
et/ou qu'on transforme une base I obtenue, par traitement avec un acide en un de ses sels et/ou qu'on obtient la forme libre à partir d'un de ses sels par traitement avec une base.

3. Procédé de préparation de compositions pharmaceutiques, caratérisé en ce que les composés I selon la revendication 1 et/ou un de leurs sels physiologiquement acceptables sont présentés sous forme de dosage approprié conjointement avec un excipient ou un adjuvant solide, liquide ou semi-liquide.

4. Compositions pharmaceutiques, caractérisées par une teneur en composés I selon la revendication 1 et/ou un de leurs sels physiologiquement acceptables.

5. Utilisation des composés I ou d'un de leurs sels physiologiquement acceptables pour la préparation d'un médicament.
